# FASCICULE DE BREVET EUROPEEN

(11) **EP 2 286 919 B1**
(45) Date de publication et mention de la délivrance du brevet: **28.06.2017**
(21) Numéro de dépôt: 10167532.0
(22) Date de dépôt: 28.06.2010
(51) Int. Cl.: B01L 3/00, B01L 3/14, A61B 10/00, G01N 1/40

(54) **Dispositif de filtration pour analyses en parasitologie**
Filtervorrichtung für Parasitologie-Analysen
Filtration device for parasitology analyses

(30) Priorité: 21.07.2009 FR 0903571
(43) Date de publication de la demande: 23.02.2011
(73) Titulaire: Groupe Servibio, 91952 Courtaboeuf CEDEX (FR)
(72) Inventeur: Gabrielli, Marco, 95170, DEUIL LA BARRE (FR)
(74) Mandataire: Gaillarde, Frédéric F. Ch.

(56) Documents cités:
- EP-A1- 0 614 675
- GB-A- 2 321 857
- GB-A- 2 433 219
- US-A- 5 779 674
- US-A- 5 882 943
- US-A- 5 925 250
- US-A1- 2008 185 349

## Description

La présente invention se rapporte à un dispositif de filtration pour analyses, notamment en parasitologie.

La présente invention trouve plus particulièrement application dans le règne animal des vertébrés ; chez l'homme, les bovins les ovins et les équidés mais n'y est pas limité. Les vers gastro-intestinaux sont présents dans tous les troupeaux de petits ruminants et leur infestation par ces parasites constitue un problème sanitaire et économique important. A un niveau clinique, la présence de parasites gastro-intestinaux affecte notamment la croissance des animaux ou leur production de lait, par exemple, plus particulièrement en réduisant leur prise alimentaire. En dessous du niveau clinique, la présence de parasites affecte la qualité de la viande et du lait.

Par ailleurs, ces vers peuvent développer une résistance à certains vermifuges, former des larves et oeufs voir des kystes (formes de résistance)

Aussi, la mise en oeuvre de mesures préventives devient de plus en plus importante et nécessaire.

Il apparaît également que dans un troupeau, seule une minorité d'animaux est généralement responsable de la contamination d'un pâturage.

La détection des infestations se base par conséquent principalement sur l'évaluation des risques, l'observation de l'état de santé général des animaux, les analyses coprologiques ainsi que sur les autopsies et contrôles effectués en abattoir.

Cet aspect est particulièrement important dans le contexte de l'agriculture biologique puisque les traitements vétérinaires des animaux doivent être évités et soigneusement contrôlés.

Le dispositif de filtration concerné par la présente invention vise à faciliter et améliorer les analyses coprologiques permettant de détecter la présence d'oeufs de parasites dans les fèces des hommes et des animaux et d'en déterminer la proportion.

Les documents GB 2 321 857 et GB 2 433 219 décrivent respectivement un dispositif de filtration conçu pour ces analyses et une méthode d'analyse utilisant ledit dispositif de filtration.

Le protocole d'analyse consiste à prélever une certaine quantité de fèces humaines ou animales dispersées dans un volume d'eau en présence d'un agent réactif concentrateur et de mise en évidence, notamment par coloration, de parasites (on se référera notamment aux techniques Bailenger, Flottasep ou Servisol par exemple), l'ensemble étant ensuite vortexé ou mélangé dans un premier flacon à fins d'émulsification de la solution.

Le mélange émulsifié est ensuite filtré et transvasé dans un second flacon dans lequel il est laissé à sédimenter. C'est l'étape de concentration des parasites

Les particules sédimentées et / ou piégées dans le filtre peuvent être retirées pour procéder à l'analyse de parasites, notamment par microscope.

Un dispositif de filtration « tout-en-un » tel que décrit et revendiqué dans le document GB 2 321 857 a permis de faciliter une telle analyse, d'améliorer les conditions d'hygiène, et d'avoir des résultats de tests plus probants et fiables.

Malgré ces avantages, il existe un besoin constant d'optimisation de la filtration. Un ultérieur dispositif de filtration est divulgué dans le document US-A-2008/0185349, correspondant au préambule de la revendication 1. Par ailleurs, les procédés d'analyse utilisent maintenant de préférence des solutions aqueuses sans solvant organique et notamment sans formol. Les matières fécales contiennent cependant des graisses qui doivent donc être retenues et piégées le plus possible par le système de filtration et ce quel que soit leur taille.

La présente invention vise à proposer un filtre tel que décrit précédemment possédant une efficacité accrue et se rapporte pour ce faire à un dispositif de filtration comprenant un corps central formant bouchon possédant une première extrémité apte à recevoir un premier flacon, et une deuxième extrémité apte à recevoir un deuxième flacon, ledit bouchon présentant un canal traversant débouchant, d'une part, au niveau de sa première extrémité à l'intérieur d'un filtre sensiblement tubulaire s'étendant depuis ladite première extrémité, et d'autre part, au niveau de la deuxième extrémité, caractérisé en ce que le filtre comprend au moins deux portions longitudinales possédant chacune une porosité différente.

Ainsi, il a été constaté que le fait de prévoir des portions de porosité différentes et adaptées le long du filtre permettait d'améliorer notablement de nombreux paramètres, notamment : la vitesse de filtration, les risques de colmatage du filtre, limiter le passage des graisses à travers le filtre.

Avantageusement, les portions de porosités différentes sont de porosité décroissante en s'éloignant de la première extrémité.

En effet, les particules de tailles importantes et les graisses restent globalement en surface du liquide à filtrer. Ainsi, les particules et les graisses sont retenues par la portion de porosité faible tandis que le liquide contenant moins de graisse et de particules est filtré plus rapidement. Le filtrat contient donc sensiblement moins de graisse qu'avec un filtre selon l'art antérieur.

De manière préférentielle, le filtre présente trois portions de porosités différentes.

Préférentiellement, chaque portion de porosité différente est sensiblement de la même longueur.

De manière avantageuse, le filtre possède au moins deux portions dont au moins une porosité est choisie parmi les valeurs suivantes : sensiblement 200µm, sensiblement 300µm et sensiblement 400µm.

De manière complémentaire avantageuse, le corps central présente au niveau de sa première extrémité et / ou au niveau de sa deuxième extrémité un filetage externe apte à coopérer avec un taraudage correspondant du flacon destiné à être rapporté sur ladite première et/ou deuxième extrémité.

Avantageusement, chaque extrémité est associée à un filetage externe correspondant, les filetages étant réalisés tournant dans le même sens. Ainsi, les sens de vissage et de dévissage sont bien à l'inverse l'un de l'autre pour chaque flacon montés en opposition l'un de l'autre.

Avantageusement encore, le filetage de la première extrémité est associée à une dent de verrouillage, notamment réalisée sous la forme d'un bourrelet périphérique, apte à coopérer avec un bourrelet périphérique interne correspondant du flacon destiné à être rapporté sur ladite extrémité, de manière à permettre un blocage du flacon nécessitant une force de déblocage supérieure à la force de nécessaire pour dévisser le flacon rapporté sur la deuxième extrémité.

De manière complémentaire, que la première extrémité et / ou la deuxième extrémité sont équipées de moyens d'emboîtement du flacon correspondant sur le corps central.

Il convient de noter que bien que présentée en lien avec les caractéristiques précédentes, ces caractéristiques peuvent être mises en oeuvre sur un dispositif de filtration selon l'art antérieur.

En complément d'un filetage externe, on notera alors que le dispositif de filtration peut accueillir sur une même extrémité des flacons de taille et volume différents, soit un flacon conçu pour s'adapter sur le filetage externe, soit un flacon conçu pour s'emmancher. Les diamètres pourront être facilement adaptés pour des flacons standards équipant les laboratoires d'analyse.

De manière complémentaire, le canal intérieur du corps central est équipé d'un second filtre s'étendant depuis la deuxième extrémité.

Avantageusement, ce second filtre est amovible.

Préférentiellement, le second filtre présente un gradient de porosité.

Préférentiellement encore, les portions de porosités différentes du second filtre sont de porosités croissantes en s'éloignant de la deuxième extrémité.

Avantageusement, le filtre tubulaire possède une section générale hexagonale, l'extrémité distale pouvant être arrondie. Ainsi, la surface globale de filtration s'en trouve augmentée par rapport à un filtre présentant une section générale sensiblement circulaire.

La présente invention se rapporte également à un ensemble de filtration caractérisé en ce qu'il comprend, d'une part, un dispositif de filtration selon l'invention, et d'autre part, un flacon rapporté sur la deuxième extrémité du dispositif de filtration.

La présente invention sera mieux comprise à la lumière de la description détaillée qui suit en regard du dessin annexé dans lequel :
- La figure 1 est une vue en perspective d'un dispositif de filtration selon l'invention.
- La figure 2 est une vue en perspective de l'intérieur du dispositif de la figure 1.
- La figure 3 est une vue en coupe longitudinale du dispositif de la figure 1.
- La figure 4 est une vue de côté du dispositif de la figure 1.
- La figure 5 est une représentation schématique en coupe transversale d'une variante de réalisation du filtre de la figure 1 équipé d'un filtre secondaire.

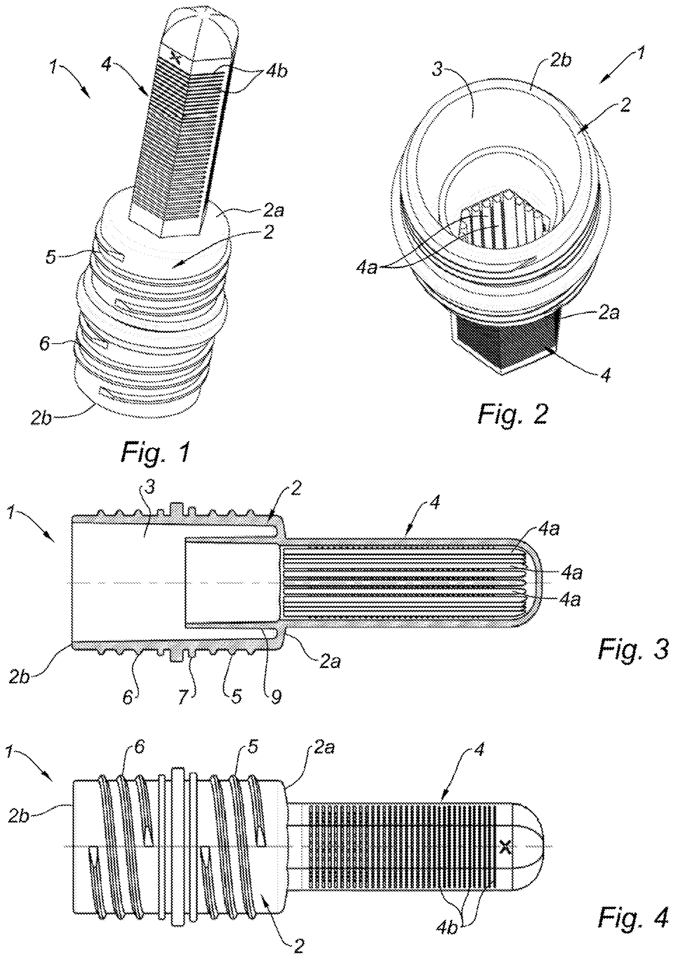

Un dispositif de filtration 1 tel que représenté sur les figures 1 à 4 comprend un corps central 2 formant bouchant possédant une première extrémité 2a apte à recevoir un premier flacon (non représenté), et une deuxième extrémité 2b apte à recevoir un deuxième flacon.

Le corps central 2 présente par ailleurs un canal intérieur 3 traversant débouchant, d'une part, au niveau de la première extrémité 2a à l'intérieur d'un filtre 4 longitudinal sensiblement tubulaire s'étendant depuis ladite première extrémité 2a, et d'autre part, au niveau de la deuxième extrémité.

L'ensemble du bouchon pourra être notamment réalisé en matière plastique injectée par exemple.

Le filtre pourra notamment être réalisé sous la forme d'un réseau de croisillons 4a, 4b sensiblement perpendiculaires entre eux et comprenant des barres longitudinales 4a et des fentes 4b ménagées dans une paroi externe périphérique du filtre 4.

La superposition des barres longitudinales 4a et des parties restantes de la paroi périphérique, permettent de limiter les risquent d'obturation des pores formés.

Selon l'invention, le filtre 4 est réalisé de manière à présenter une pluralité de portions filtrantes présentant des porosités différentes.

Plus précisément, le filtre représenté possède trois portions possédant respectivement des porosités de 400 µm, 300µm et 200µm. Chaque portion filtrante a sensiblement la même longueur.

On notera que la portion de plus grande porosité sera située préférentiellement à proximité de la première extrémité 2a, tandis que la portion de plus faible porosité sera située à une extrémité distale du filtre 4.

Afin d'augmenter la surface filtrante, le filtre 4 longitudinale présente une section générale sensiblement hexagonale. Son extrémité distale est toutefois arrondie.

L'installation des flacons sur le corps central 2 s'effectue par le biais de filetages externes 5, 6 équipant sa première 2a et sa deuxième extrémité 2b et destinés chacun à coopérer avec un taraudage correspondant dudit flacon.

On notera que les filetages 5, 6 sont réalisés tournant dans le même sens autour du corps central 2. Ainsi les mouvements de vissage et de dévissage des flacons montés en opposition l'un l'autre sont inverses.

De plus, afin de sécuriser le flacon contenant l'échantillon de selles (flacon rapporté sur la première extrémité 2a et monté sur le filtre 4), il est également prévu un cliquet ou une dent de blocage réalisée sous la forme d'un bourrelet périphérique 7 situé légèrement après la fin du filetage 5. Ce bourrelet 7 est destiné à coopérer avec un bourrelet périphérique intérieur du flacon (non représenté). Ainsi, la force nécessaire au désengagement du bourrelet du flacon d'avec le bourrelet périphérique 7 est supérieur à la force nécessaire pour dévisser le second flacon, et supprime ainsi un dévissage malencontreux du premier flacon.

Dans le but de présenter un caractère adaptable à de multiples taille de flacon, il est prévu des moyen d'emboîtement associés à la deuxième extrémité.

Ces moyens se présentent sous la forme d'un col intérieur 9 conçu pour permettre le maintien du rebord d'un flacon introduit entre ledit col 9 et la paroi du corps central 2.

On pourra ainsi au choix adapter un flacon d'un diamètre d'environ 15 mm emmanché dans la deuxième extrémité 2b ou un flacon d'un diamètre d'environ 29 mm, par exemple, sur le filetage externe en fonction de la quantité d'échantillon prélevée et le volume à filtrer.

Ces tailles de flacons sont courantes dans les laboratoires d'analyses et il n'est donc pas nécessaire de prévoir des flacons spécifiques.

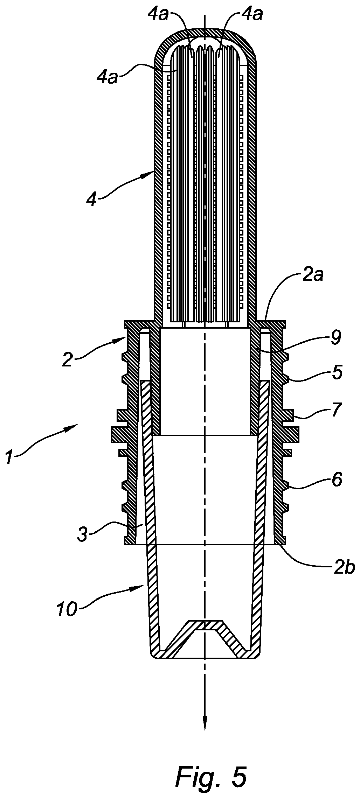

En outre, conformément au mode de réalisation représenté sur la figure 5, le col intérieur 9 pourra être utilisé pour installer un filtre secondaire 11 s'étendant depuis la deuxième extrémité présentant une forme sensiblement tronconique.

On notera que le mode de réalisation de la figure 5 présente un filtre 1 équipé d'un filtre secondaire 10 rapporté sur le col intérieur 9. Le filtre secondaire pourra ainsi être rapporté au besoin selon l'échantillon à filtrer. Ainsi, le dispositif de filtration 1, notamment grâce à ce col intérieur 9, pourra toujours recevoir des tubes de tailles différentes. Bien évidemment, on pourra prévoir un filtre secondaire 10 intégré au dispositif de filtration 1.

Ce filtre secondaire permet d'améliorer encore la rétention des graisses et particules pouvant encore être présentes dans le filtrat issu du premier filtre 4.

La porosité du premier filtre étant comprise entre 200 et 400 microns selon le mode de réalisation préféré, donné en exemple, le second filtre présentera avantageusement une porosité comprise entre environ 150 et 300 microns.

Avantageusement, ce second filtre 10 présentera, comme le premier filtre un gradient de porosité avec des porosités comprises notamment entre 150 et 300 microns et disposées de manière croissantes en s'éloignant de la deuxième extrémité.

Ainsi, on notera que les ordres de porosités du filtre principal 4 et du filtre secondaire 10 sont inversés (flux de filtration de haut en bas sur la figure 5).

En effet, la solution initiale étant centrifugée dans le tube au début du protocole d'analyse, les graisses peuvent en partie s'émulsionner et donc se présenter sous forme de particules de très petites tailles.

Ces particules de très petites tailles sont donc susceptibles de traverser le filtre principal 4. Le filtre secondaire 10 permet de faire subir au premier filtrat une deuxième filtration visant à réduire encore la quantité de graisses dans la solution finale, notamment en permettant aux particules de graisses émulsionnées de se réagglomérer et d'être retenues dans le second filtre 10.

Comme le premier filtre 4, le second filtre pourra présenter une structure tridimensionnelle, et être notamment réalisé sous la forme d'un réseau de croisillons sensiblement perpendiculaires entre eux et comprenant des barres longitudinales et des fentes ménagées dans une paroi externe périphérique du filtre 10. Un tel filtre 10 tridimensionnel permet de réduire fortement le risque de bouchage des ouvertures du filtre 10 par les particules graisseuses.

Grâce à ce second filtre, et conformément à un objectif fondamental de l'invention; le dispositif de filtration permet d'obtenir une élimination totale de graisses dans l'échantillon à analyser.

Le recours à des solvants organiques usuels, tels que l'éther, le formol, l'acétate d'éthyle, n'est donc plus nécessaire, ce qui représente un intérêt écologique majeur tant pour la protection de l'environnement que pour le traitement des déchets constitués et imprégnés de ces mêmes solvants. Par ailleurs, l'intégrité biologique de l'échantillon est conservée.

C'est donc un ensemble filtre primaire et filtre secondaire en porosité inversée qui est mis en exergue pour stopper toute présence de particules graisseuses dans le filtrat obtenu en chambre pour mener l'étude d'identification des oeufs larves et kystes sans interférences de lecture.

## Revendications

1. Dispositif de filtration (1) pour analyses en parasitologie, comprenant un corps central (2) formant bouchon possédant une première extrémité (2a) apte à recevoir un premier flacon, et une deuxième extrémité (2b) apte à recevoir un deuxième flacon, ledit bouchon présentant un canal traversant (3) débouchant, d'une part, au niveau de sa première extrémité à l'intérieur d'un filtre (4) tubulaire s'étendant depuis ladite première extrémité, et d'autre part, au niveau de la deuxième extrémité, **caractérisé en ce que** le filtre comprend au moins deux portions longitudinales possédant chacune une porosité différente.

2. Dispositif (1) selon la revendication 1, **caractérisé en ce que** les portions de porosités différentes sont de porosités décroissantes en s'éloignant de la première extrémité.

3. Dispositif (1) selon l'une quelconque des revendications 1 ou 2, **caractérisé en ce que** le filtre (4) présente trois portions de porosités différentes.

4. Dispositif (1) selon l'une quelconque des revendications 1 à 3, **caractérisé en ce que** chaque portion de porosité différente est de la même longueur.

5. Dispositif (1) selon l'une quelconque des revendications 1 à 4, **caractérisé en ce que** le filtre (4) possède au moins deux portions dont au moins une porosité est choisie parmi les valeurs suivantes : 200µm, 300µm et 400µm.

6. Dispositif (1) selon l'une quelconque des revendications 1 à 5, **caractérisé en ce que** le corps central (2) présente au niveau de sa première extrémité (2a) et / ou au niveau de sa deuxième extrémité (2b) un filetage (5, 6) externe apte à coopérer avec un taraudage correspondant du flacon destiné à être rapporté sur ladite première et/ou deuxième extrémité.

7. Dispositif (1) selon la revendication 6, **caractérisé en ce que** chaque extrémité (2a, 2b) est associée à un filetage externe (5, 6) correspondant, les filetages étant réalisés tournant dans le même sens.

8. Dispositif (1) selon l'une quelconque des revendications 6 ou 7, **caractérisé en ce que** le filetage (5) de la première extrémité (2a) est associée à une dent de verrouillage, notamment réalisée sous la forme d'un bourrelet périphérique (7), apte à coopérer avec un bourrelet périphérique interne correspondant du flacon destiné à être rapporté sur ladite extrémité, de manière à permettre un blocage du flacon nécessitant une force de déblocage supérieure à la force de nécessaire pour dévisser le flacon rapporté sur la deuxième extrémité (2b).

9. Dispositif (1) selon l'une quelconque des revendications 1 à 8, **caractérisé en ce que** la première extrémité (2a) et / ou la deuxième extrémité (2b) sont associés à des moyens d'emboîtement du flacon correspondant sur le corps central (2).

10. Dispositif (1) selon l'une quelconque des revendications 1 à 9, **caractérisé en ce que** le canal intérieur (3) du corps central (2) est équipé d'un second filtre s'étendant depuis la deuxième extrémité (2b).

11. Dispositif selon la revendication 10, **caractérisé en ce que** le second filtre (10) est amovible.

12. Dispositif selon l'une quelconque des revendications 10 ou 11, **caractérisé en ce que** le second filtre (10) présente un gradient de porosité.

13. Dispositif selon la revendication 12, **caractérisé en ce que** les portions de porosités différentes du second filtre (10) sont de porosités croissantes en s'éloignant de la deuxième extrémité (2b).

14. Dispositif (1) selon l'une quelconque des revendications 1 à 13, **caractérisé en ce que** le filtre tubulaire (4) possède une section générale hexagonale, l'extrémité distale pouvant être arrondie.

15. Ensemble de filtration **caractérisé en ce qu'**il comprend, d'une part, un dispositif de filtration (1) selon l'une quelconque des revendications 1 à 14, et d'autre part, un flacon rapporté sur la deuxième extrémité (2b) du dispositif de filtration.

## Patentansprüche

1. Filtervorrichtung (1) für Analysen in der Parasitologie, umfassend einen zentralen Körper (2), der einen Stopfen bildet, der ein erstes Ende (2a) aufweist, das ausgelegt ist, um einen ersten Kolben aufzunehmen, und ein zweites Ende (2b), das ausgelegt ist, um einen zweiten Kolben aufzunehmen, wobei der Stopfen einen Querkanal (3) aufweist, der einerseits auf der Ebene seines ersten Endes im Inneren eines rohrförmigen Filters (4) mündet, der sich von dem ersten Ende erstreckt, und andererseits auf der Ebene des zweiten Endes, **dadurch gekennzeichnet, dass** der Filter mindestens zwei Längsabschnitte umfasst, die jeweils eine verschiedene Porosität aufweisen.

2. Vorrichtung (1) nach Anspruch 1, **dadurch gekennzeichnet, dass** die Abschnitte mit verschiedenen Porositäten von abnehmenden Porositäten sind, wenn sie sich vom ersten Ende entfernen.

3. Vorrichtung (1) nach einem der Ansprüche 1 oder 2, **dadurch gekennzeichnet, dass** der Filter (4) drei Abschnitte mit verschiedenen Porositäten aufweist.

4. Vorrichtung (1) nach einem der Ansprüche 1 bis 3, **dadurch gekennzeichnet, dass** jeder Abschnitt mit verschiedener Porosität der gleichen Länge ist.

5. Vorrichtung (1) nach einem der Ansprüche 1 bis 4, **dadurch gekennzeichnet, dass** der Filter (4) mindestens zwei Abschnitte aufweist, von denen mindestens eine Porosität ausgewählt ist aus den folgenden Werten: 200 µm, 300 µm und 400 µm.

6. Vorrichtung (1) nach einem der Ansprüche 1 bis 5, **dadurch gekennzeichnet, dass** der zentrale Körper (2) auf der Ebene seines ersten Endes (2a) und/oder auf der Ebene seines zweiten Endes (2b) ein Gewinde (5, 6) aufweist, das ausgelegt ist, um mit einem entsprechenden Innengewinde des Kolbens zusammenzuarbeiten, der dazu vorgesehen ist, auf das erste und/oder das zweite Ende aufgesetzt zu werden.

7. Vorrichtung (1) nach Anspruch 6, **dadurch gekennzeichnet, dass** jedes Ende (2a, 2b) mit einem entsprechenden äußeren Gewinde (5, 6) assoziiert ist, wobei die Gewinde in die gleiche Richtung drehend durchgeführt sind.

8. Vorrichtung (1) nach einem der Ansprüche 6 oder 7, **dadurch gekennzeichnet, dass** das Gewinde (5) des ersten Endes (2a) mit einem Verriegelungszahn assoziiert ist, der insbesondere in Form eines Umfangsdichtungsstreifens (7) durchgeführt ist, der ausgelegt ist, um mit einem entsprechenden inneren Umfangsdichtungsstreifen des Kolbens zusammenzuarbeiten, der dazu vorgesehen ist, auf das Ende aufgesetzt zu werden, um eine Blockierung des Kolbens zu ermöglichen, die eine Entblockierungskraft erfordert, die höher als die Kraft ist, die erforderlich ist, um den Kolben abzuschrauben, der auf das zweite Ende (2b) aufgesetzt ist.

9. Vorrichtung (1) nach einem der Ansprüche 1 bis 8, **dadurch gekennzeichnet, dass** das erste Ende (2a) und/oder das zweite Ende (2b) mit Einschubmitteln des entsprechenden Kolbens auf den zentralen Körper (2) assoziiert sind.

10. Vorrichtung (1) nach einem der Ansprüche 1 bis 9, **dadurch gekennzeichnet, dass** der innere Kanal (3) des zentralen Körpers (2) mit einem zweiten Filter ausgestattet ist, der sich vom zweiten Ende (2b) erstreckt.

11. Vorrichtung nach Anspruch 10, **dadurch gekennzeichnet, dass** der zweite Filter (10) abnehmbar ist.

12. Vorrichtung nach einem der Ansprüche 10 oder 11, **dadurch gekennzeichnet, dass** der zweite Filter (10) einen Porositätsgradienten aufweist.

13. Vorrichtung nach Anspruch 12, **dadurch gekennzeichnet, dass** die Abschnitte mit verschiedenen Porositäten des zweiten Filters (10) von zunehmenden Porositäten sind, wenn sie sich vom zweiten Ende (2b) entfernen.

14. Vorrichtung (1) nach einem der Ansprüche 1 bis 13, **dadurch gekennzeichnet, dass** der rohrförmige Filter (4) einen allgemeinen Abschnitt aufweist.

15. Filtereinheit, **dadurch gekennzeichnet, dass** sie einerseits eine Filtervorrichtung (1) nach einem der Ansprüche 1 oder 14, umfasst, und andererseits einen Kolben, der auf das zweite Ende (2b) der Filtervorrichtung aufgesetzt ist.

## Claims

1. A filtration device (1) for analyzes in parasitology, comprising a central body (2) forming a plug having a first end (2a) capable of receiving a first vial, and a second end (2b) capable of receiving a second vial, said plug having a through channel (3) opening, on the one hand, at its first end inside a tubular filter (4) extending from said first end and, on the other hand, at the second end, **characterized in that** the filter comprises at least two longitudinal portions each having a different porosity.

2. The device (1) according to claim 1, **characterized in that** the portions of different porosities have decreasing porosities while becoming more distant from the first end.

3. The device (1) according to any one of claims 1 or 2, **characterized in that** the filter (4) has three portions of different porosities.

4. The device (1) according to any one of claims 1 to 3, **characterized in that** each portion of different porosity has the same length.

5. The device (1) according to any one of claims 1 to 4, **characterized in that** the filter (4) has at least two portions whose at least one porosity is chosen from the following values: 200µm, 300µm and 400µm.

6. The device (1) according to any one of claims 1 to 5, **characterized in that** the central body (2) has at its first end (2a) and/or at its second end (2b) an outer thread (5, 6) capable of cooperating with a corresponding tapping of the vial intended to be attached on said first and/or second end.

7. The device (1) according to claim 6, **characterized in that** each end (2a, 2b) is associated with a corresponding outer thread (5, 6), the threads being made rotating in the same direction.

8. The device (1) according to any one of claims 6 or 7, **characterized in that** the thread (5) of the first end (2a) is associated with a locking tooth, in particular made in the form of a peripheral bead (7), capable of cooperating with a corresponding inner peripheral bead of the vial intended to be attached on said end, so as to allow a blocking of the vial requiring an unblocking force greater than the force necessary to unscrew the vial attached on the second end (2b).

9. The device (1) according to any one of claims 1 to 8, **characterized in that** the first end (2a) and/or the second end (2b) are associated with means for interlocking the corresponding vial onto the central body (2).

10. The device (1) according to any one of claims 1 to 9, **characterized in that** the inner channel (3) of the central body (2) is equipped with a second filter extending from the second end (2b).

11. The device according to claim 10, **characterized in that** the second filter (10) is removable.

12. The device according to any one of claims 10 or 11, **characterized in that** the second filter (10) has a porosity gradient.

13. The device according to claim 12, **characterized in that** the portions of different porosities of the second filter (10) have increasing porosities while becoming more distant from the second end (2b).

14. The device (1) according to any one of claims 1 to 13, **characterized in that** the tubular filter (4) has a general hexagonal section, the distal end can be rounded.

15. A filtration assembly **characterized in that** it comprises, on the one hand, a filtration device (1) according to any one of claims 1 to 14 and, on the other hand, a vial attached on the second end (2b) of the filtration device.
